# EUROPEAN PATENT APPLICATION

(11) **EP 2 891 504 A1**
(43) Date of publication of application: **08.07.2015**
(21) Application number: 12877600.2
(22) Date of filing: 31.12.2012
(51) Int. Cl.: A61M 5/32

(54) **SAFETY INJECTION NEEDLE JACKET**

(30) Priority: 31.08.2012 CN 201220438936 U
(71) Applicant: Zhejiang Kindly Medical Devices Co., Ltd., Yongzhong, Longwan District Wenzhou Zhejiang (CN)
(72) Inventor: ZHANG, Qian, Zhejiang 325024 (CN); CHEN, Hong, Zhejiang 325024 (CN)
(74) Representative: Chaillot, Geneviève
(86) International application number: PCT/CN2012/088027
(87) International publication number: WO 2014/032383

(57) **Abstract**

A safety needle assembly includes a needle (1) and a protection cover (2). The needle (1) is composed of a needle seat (101) and a needle body (102). The protection cover (2) has a needle accommodation room (201) therein. The needle accommodation room (201) has a barb (202) therein. One side of a lower end of the protection cover (2) is rotatable and connected to the needle seat (101). The barb (202) is disposed in the needle accommodation room (201) and integrally formed with the protection cover (2). A buckle mechanism is provided between the side wall of the needle accommodation room (201) and the needle seat (101). The protection cover (2) is directly connected to the needle seat (101), without a protection cover seat. The barb (202) is integrally formed with the protection cover (2). The structure simpler and it is easy to be produced. The buckle mechanism is provided between the side wall of the needle accommodation room (201) and the needle seat (101). The protection cover (2) and the needle (1) can be buckled reliably and safely.

## Description

### BACKGROUND OF THE INVENTION

### 1. Field of the Invention

The present invention relates to a medical apparatus, and more particularly to a safety needle assembly.

### 2. Description of the Prior Art

A conventional needle assembly is composed of a needle body, a needle seat, a connecting portion and a protection cover. The needle body and the needle seat are connected through the connecting portion. The protection cover is fitted on the outer wall of the needle seat to protect the needle body before the needle is used and to prevent a person being hurt by accident. When in use, the needle is inserted and fixed to the awl head of a syringe through the inner hole of the needle seat. Most users throw away the protection cover. After that, the user proceeds to draw drug and inject the drug. After injection, some people may use the protection cover to cover the needle. This way is not safe for use. Sometimes, people may be hurt by accident. Besides, it is a burden to dispose medical waste. In order to solve these problems, the applicant invented a safety needle protection cover as disclosed in Chinese Utility Model Application Number 200920215511.X filed Dec. 30, 2009. The safety needle protection cover comprises a protection cover base and a protection cover. The protection cover base has a cover hole mating with the needle. One side of the lower end of the protection cover is rotatably connected to the protection cover base. The protection cover has a needle accommodation room therein. The protection cover is disposed relative to the lower opening of the accommodation room. The protection cover is hingedly connected with a rotatable lock handle close to the side wall of the accommodation room. The inner end of the rotatable lock handle is a hook. The hook includes a rear board and a barb connected to the lower end of the rear board. The side wall of the accommodation room of the protection cover, relative to the rear board, is provided with two front and rear lock protrusions for engagement of the rear board. The outer end of the rotatable lock handle is a reversal lever. The protection cover has a through hole relative to the top wall of the accommodation room to mate with the reversal lever. The outer end of the reversal lever passes the through hole and stresses the protection cover base when the protection cover base and the protection cover are relatively turned. Although this safety needle protection cover can cover the needle after use to prevent people from being hurt, it still has some shortcomings. The needle is only held by the barb so it is not reliable. The rotatable lock handle and the protection cover are separate. The rotatable lock handle is connected to the protection cover through a pin. The production and assembly are not convenient. It is necessary to provide the protection cover base so the structure is complicated and the material and the cost increase. Accordingly, the inventor of the present invention has devoted himself based on his many years of practical experiences to solve this problem.

### SUMMARY OF THE INVENTION

The primary object of the present invention is to provide a safety needle assembly to overcome the shortcomings of the prior art. The safety needle is cost-effective and can be produced easily and is used safely and reliably.

In order to achieve the aforesaid object, the safety needle assembly of the present invention comprises a needle and a protection cover. The needle is composed of a needle seat and a needle body. The protection cover has a needle accommodation room therein. The needle accommodation room has a barb therein. One side of the lower end of the protection cover is rotatable and connected to the needle seat. The barb is disposed in the needle accommodation room and integrally formed with the protection cover. A buckle mechanism is provided between the side wall of the needle accommodation room and the needle seat.

Compared to the prior art, the protection cover of the present invention is directly connected to the needle seat, without a protection cover seat. The barb is integrally formed with the protection cover. The structure simpler and it is easy to be produced. The buckle mechanism is provided between the side wall of the needle accommodation room and the needle seat. The protection cover and the needle can be buckled reliably and safely.

### BRIEF DESCRIPTION OF THE DRAWINGS

Fig. 1 is a schematic view according to a preferred embodiment of the present invention when in use;
Fig. 2 is another schematic view according to the preferred embodiment of the present invention when in use;
Fig. 3 is a side view according to the preferred embodiment of the present invention after use;
Fig. 4 is a front view according to the preferred embodiment of the present invention after use;
Fig. 5 is a rear view according to the preferred embodiment of the present invention after use;
Fig. 6 is a sectional view taken along line A-A of Fig. 5; and
Fig. 7 is a sectional view taken along line B-B of Fig. 5.

### DETAILED DESCRIPTION OF THE PREFERRED EMBODIMENTS

Embodiments of the present invention will now be described, by way of example only, with reference to the accompanying drawings.

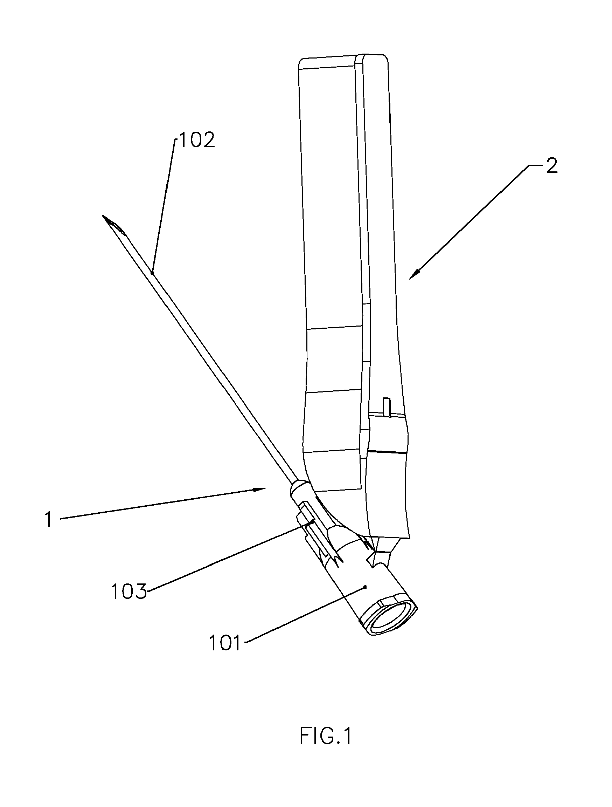

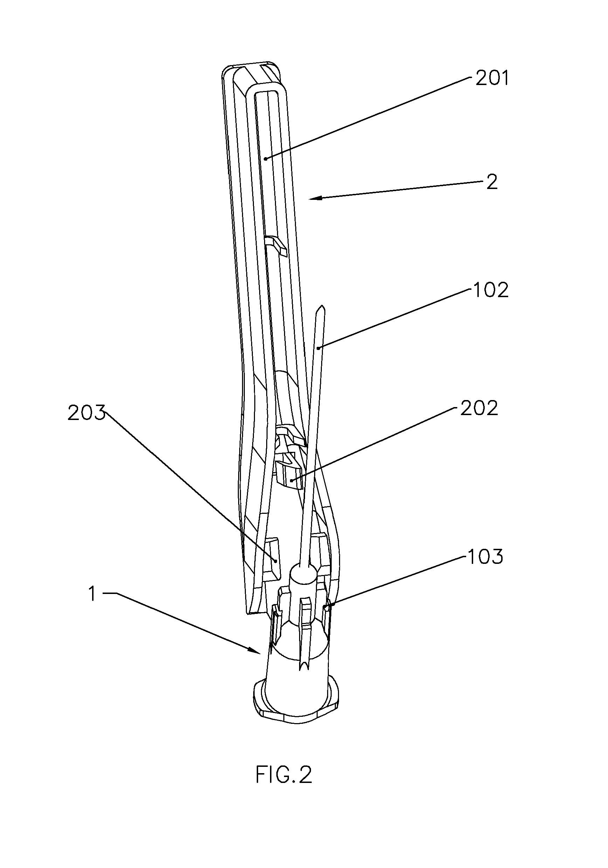

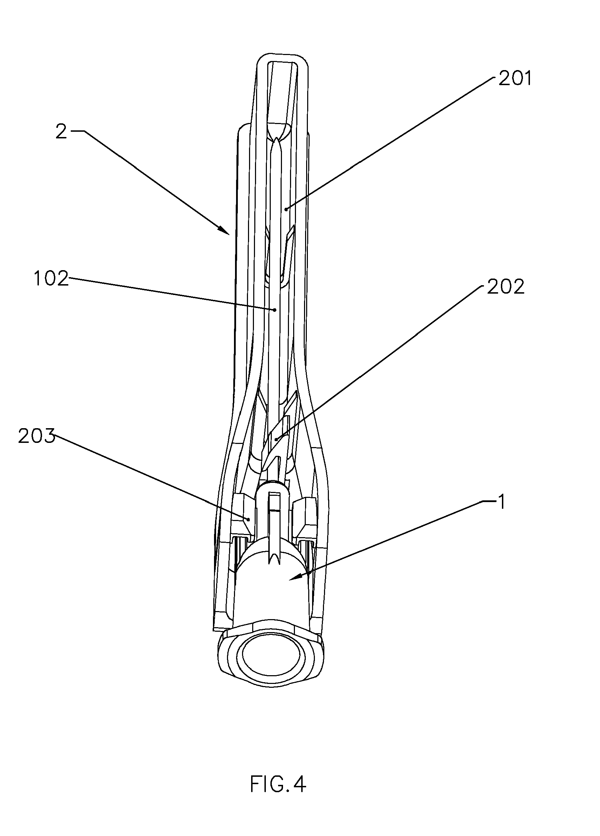

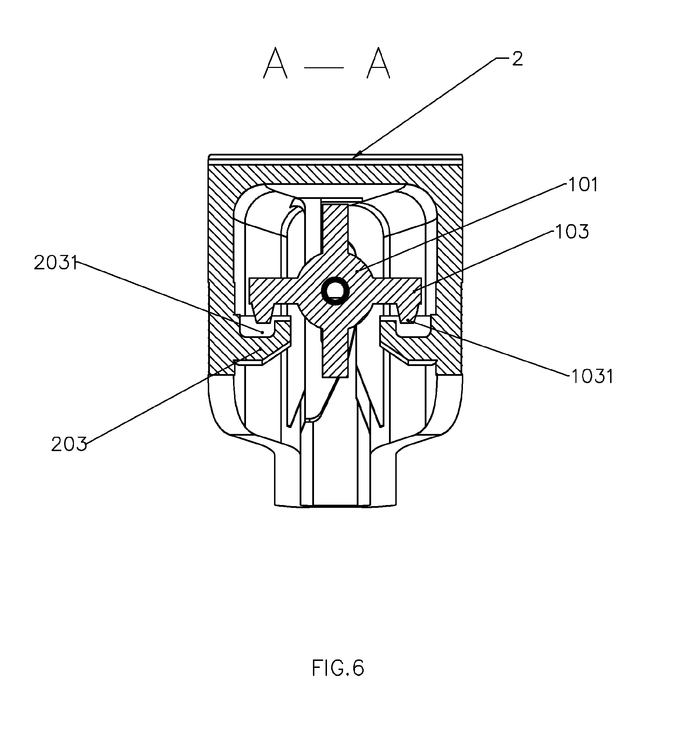

As shown in Fig. 1 and Fig. 2, the safety needle assembly according to a preferred embodiment of the present invention comprises a needle 1 and a protection cover 2. The needle 1 is composed of a needle seat 101 and a needle body 102. The protection cover 2 corresponds in length to the needle 1. The protection cover 2 has an opening at one side thereof, with a U-shaped cross-section to form a needle accommodation room 201. The length and width of the needle accommodation room 201 is adapted to accommodate the needle seat 101 and the needle body 102. One side of the lower end of the protection cover 2 is rotatable and connected to the outer wall of the needle seat 101. The opening of the needle accommodation room 201 faces the needle body 102, so that the needle seat 101 and the needle body 102 can rotated to be received in the needle accommodation room 201. The connection way can adopt an integral injection by using the property of plastic. The needle accommodation room 201 is integrally formed with a barb 202. The barb 202 is fixed to the bottom wall of the needle accommodation room 201. A buckle mechanism is provided between the side wall of the needle accommodation room 201 and the needle seat 101. In an embodiment, the buckle mechanism comprises an engaging block 103 provided on one side of the needle seat 101 relative to the side wall of the needle accommodation room 201 and an engaging hook 203 provided on the corresponding side wall of the needle accommodation room 201. As shown in Fig. 4 and Fig. 6, preferably, two side walls of the needle accommodation room 201 are provided with two symmetrical engaging hooks 203, and the need seat 101 is provided with two engaging blocks 103 corresponding in position to the engaging hooks 203. This way is more reliable. The engaging blocks 103 are substantially strip protrusions which are disposed axially and symmetrically at two sides of the joint of the protection cover 2 and the needle seat 101. One side of the engaging block 103, facing away from the protection cover 2, has a protruding lock rib 1031. The engaging hooks 203 on the two side walls of the needle accommodation room 201 are two opposite protrusions. One side of the protrusion, facing the opening, is disposed obliquely. Another side of the protrusion, facing away from the opening, has a recess 2031 mating with the lock rib 1031. The distance between the two recesses 2031 of the two protrusions is equal to the distance between the two lock ribs 1031.

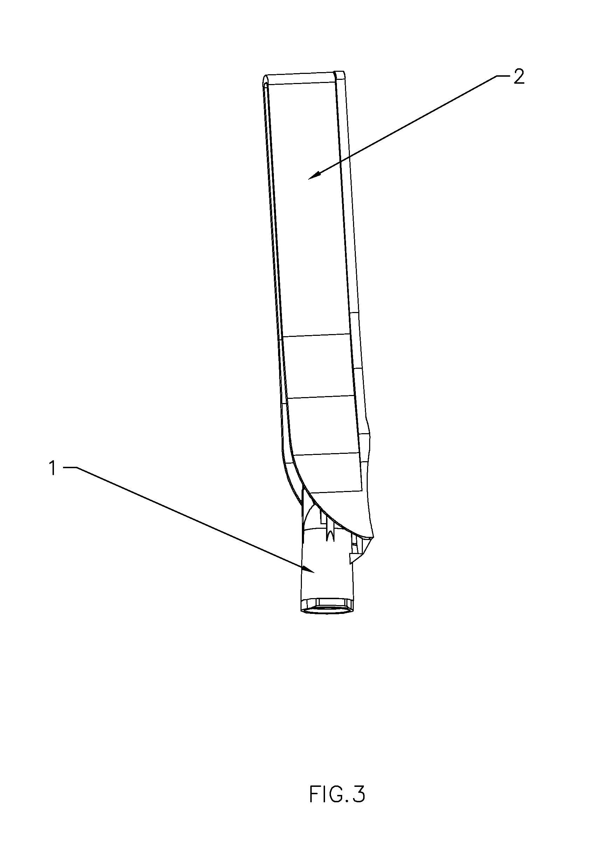

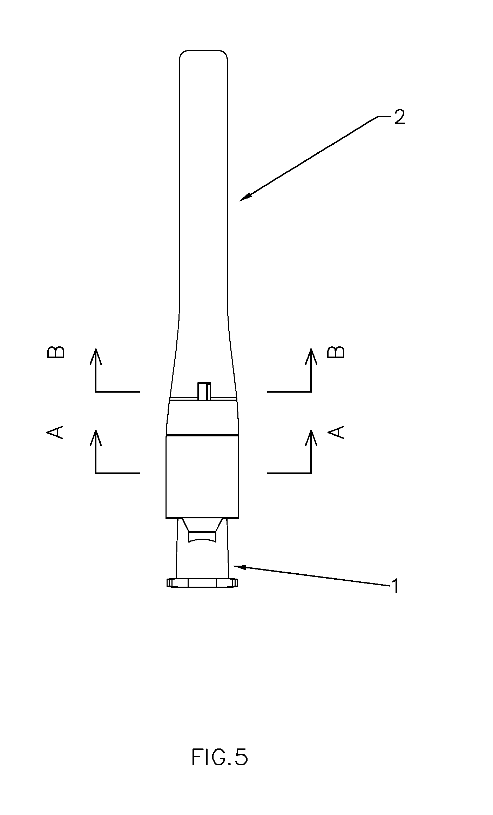

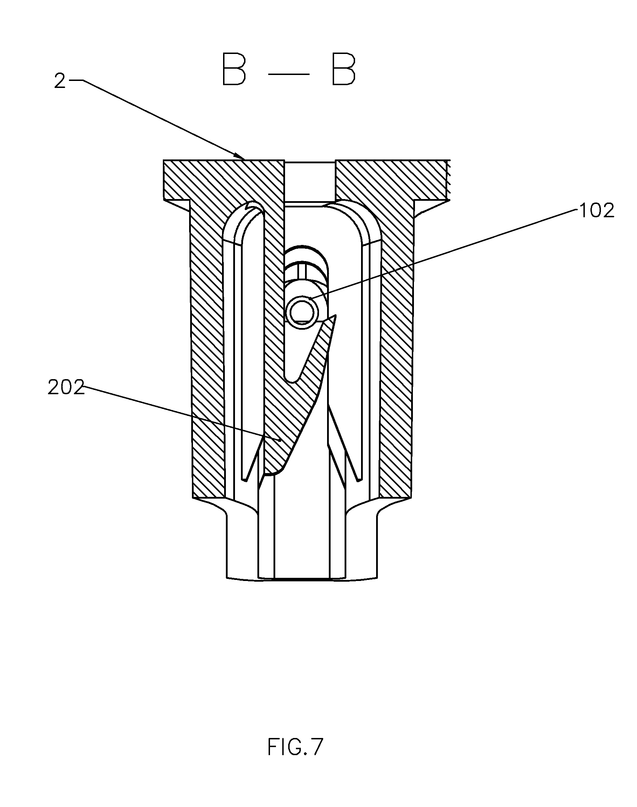

As shown in Fig. 3 to Fig. 7, after the needle 1 is used, the protection cover 2 is turned toward the needle 1 until the barb 202 hooks the needle body 102. During this procedure, the oblique surfaces of the engaging hooks 203 at the two side walls of the opening of the needle accommodation chamber 201 and the engaging blocks 103 on the needle seat 101 interact each other, so that the two side walls of the opening of the needle accommodation chamber 201 are apart from each other. Due to the design of the opening of the needle accommodation room 201, the needle accommodation room 201 is deformable. After the engaging hooks 203 slide over the engaging blocks 103, the two side walls of the needle accommodation room 201 are restored. After the engaging blocks 103 slide over the engaging hooks 203, the lock ribs 1031 on the engaging blocks 103 will be engaged in the recesses 2031 of the engaging hooks 203, so that the needle 1 and the protection cover 2 are locked each other.

Although particular embodiments of the present invention have been described in detail for purposes of illustration, various modifications and enhancements may be made without departing from the spirit and scope of the present invention. Accordingly, the present invention is not to be limited except as by the appended claims.

## Claims

1. A safety needle assembly, comprising a needle (1) and a protection cover (2), the needle (1) being composed of a needle seat (101) and a needle body (102), the protection cover (2) having a needle accommodation room (201) therein, the needle accommodation room (201) having a barb (202) therein, one side of a lower end of the protection cover (2) being rotatable and connected to the needle seat (101), the barb (202) being disposed in the needle accommodation room (201) and integrally formed with the protection cover (2), a buckle mechanism being provided between a side wall of the needle accommodation room (201) and the needle seat (101).

2. The safety needle assembly as claimed in claim 1, wherein the buckle mechanism comprises an engaging block (103) provided on one side of the needle seat (101) relative to the side wall of the needle accommodation room (201) and an engaging hook (203) provided on the corresponding side wall of the needle accommodation room (201).

3. The safety needle assembly as claimed in claim 2, wherein two side walls of the needle accommodation room (201) are provided with two symmetrical engaging hooks (203), and the need seat (101) is provided with two engaging blocks (103) corresponding in position to the engaging hooks (203).
